# EUROPEAN PATENT SPECIFICATION

(11) **EP 4 052 684 B1**
(45) Date of publication and mention of the grant of the patent: **28.01.2026**
(21) Application number: 22159634.9
(22) Date of filing: 02.03.2022
(51) Int. Cl.: A61F 2/46

(54) **INSTRUMENT AND SYSTEM FOR CONDYLAR KNEE REPLACEMENT PROCEDURES**
INSTRUMENT UND SYSTEM FÜR KONDYLÄRE KNIEERSATZEINGRIFFE
INSTRUMENT ET SYSTÈME POUR PROCÉDURES DE REMPLACEMENT CONDYLIEN DU GENOU

(30) Priority: 02.03.2021 GB 202102955
(43) Date of publication of application: 07.09.2022
(73) Proprietor: DePuy Ireland Unlimited Company, Ringaskiddy, County Cork (IE)
(72) Inventor: Brewerton, James, Leeds, LS11 8DT (GB); Amesbury, Kevin, Leeds, LS11 8DT (GB); Reason, Mark, Leeds, LS11 8DT (GB); Booth, Kevin, Leeds, LS11 8DT (GB); Meggett, Philip, Leeds, LS11 8DT (GB)
(74) Representative: Murgitroyd & Company

(56) References cited:
- DE-A1- 10 013 331
- US-A- 5 059 196
- US-A1- 2015 051 606
- US-A1- 2017 281 365

## Description

### TECHNICAL FIELD

This disclosure is concerned with the presentation and application of an implant and/or a trial implant to a surgical site, for instance in knee arthroplasty. The disclosure includes surgical instruments for such purposes and kits using such surgical instruments.

### BACKGROUND

In knee arthroplasty, the anatomic reconstruction of the joint is sought. The natural end of a bone is removed and replaced with an implant to provide a new load bearing surface. In determining the correct implant to use for a given patient, there is a need to present to the end of a bone a trial version of the implant and see if it is the most suitable size and/or geometry. The trial implant needs to be attached to a surgical instrument, detached again when in the test position and then removed again after testing. Multiple trial implants may need to be manipulated in this way. Once selected, the actual implant must also be presented and implanted in this way.

A femoral introducer is an example of such a surgical instrument. As well as positioning and releasing the implant, the femoral introducer needs to convey impact forces to the implant to assist in its positioning.

Existing designs provide for attachment, positioning, conveying of impacts and detachment, but there can be issues with the stability of the attachment to the implant or trial, particularly at one or both extremes of the size ranges and/or geometries which need to be allowed for. US2017/281365 provides a prior art system according to the claim pre-amble. US5059196, DE10013331 and US2015/051606 provide examples of other tools.

It is desirable for the surgical instrument, system or methods of use to attach and/or detach from the implant and/or trial quickly and efficiently. It is desirable for the surgical instrument, system or methods of use to provide a more stable connection during use. It is desirable for the surgical instrument, system or method of use to achieve a stable connection for a wide range of geometries and/or sizes of implant and/or trials.

### SUMMARY

An instrument according to the invention is defined in claim 1 and a kit comprising the instrument is defined in claim 11.

The casing of the instrument may include one or more slots at the distal end. The one or more slots may be provided in the sides of the casing, for instance one in each side wall, The one or more slots may provide for the first state position and the second state position of one or more of the attachment elements, for instance the one or more slots may provide for rotation of one or more of the attachment elements.

The casing proximal end may include or be a planar surface. The casing proximal end may provide an impact surface.

The casing distal end provide a mounting location or part of a mounting location. The casing distal end may provide an abutment surface, such as an implant and/or trial implant abutment surface. The casing distal end may, in use, abut a load bearing surface of an implant. The casing distal end may, in use, abut an anterior surface of the implant and/or trial implant. The casing distal end may, in use, convey impacts to the implant and/or trial implant. The casing distal end may have a profile which corresponds to at least a part of a profile of an implant and/or trial implant.

The instrument may provide a user interface. The user interface may be moveable between a first position, such as a first state, and a second position, such as a depressed state. The instrument may provide further include a lever, for instance as a user interface.

The user interface may be operably connected to the drive element.

The lever may be pivotally mounted relative to the casing. The lever may be connected to a shaft[s] that is pivotally mounted on the casing, for instance on the side of the casing. The lever may be connected to a lever body. The lever body may be connected to the shaft[s].

The shaft or shafts may extend transverse to the longitudinal axis of the casing and/or transverse to the operable direction of drive element.

The casing may include an opening for access to the lever and/or for the connection of the lever to the drive element.

The lever may have a depressed state, particularly when the attachment elements have a first state position. The lever may be flush with the outside of the casing in the depressed state. The lever may have an elevated state, particularly when the attachment elements have a second state position. The lever may project from the outside of the casing in the elevated state.

The drive element may be a rod or stem. The drive element may have a greater cross-section towards the proximal end than towards the distal end. The drive element may provide a mount for a biasing unit, such as a spring, particularly a compression spring. The mount may be provided towards the proximal end of the drive element. Alternatively, the mount may be provided towards the distal end of the drive element, for instance where a tension spring is used. The drive element may provide one or more contacts, such as drive projections, for linking the drive element to the user interface. The one or more contacts may be provided at an intermediate position relative to the distal end and the proximal end of the drive element. The drive element may provide a connection for linking the drive element to the attachment unit. The connection may be provided towards the distal end of the drive element, for instance and the distal end od the drive element. The connection may comprise an aperture in the drive element which cooperates with a pin. The pin may also cooperate with the attachment unit.

The slidable mounting for the drive element in the case may be in a bore. The bore may have a first cross-sectional area, relative to the axis of the bore, which is greater in a first area than in a second area. The second area may be closer to the proximal end of the casing than the first area.

A spring may be provided in a part of the cavity, such as a part of a bore. The second area may include a spring, for instance a compression spring. The spring, preferably the distal part thereof, may be connected to the casing or abut a part of the casing. The spring, preferably the proximal part thereof, may be connected to the driving element or abut a part of the driving element, for instance a flange. The flange may be provided at or towards the proximal end of the driving element. The spring may resist distal movement of the drive element. The spring may bias the drive element towards the proximal end of the casing. The spring may bias the lever towards an elevated state. The spring may bias the attachment elements towards a second state position.

The drive element may pass through a part of the lever body. The drive element may pass through a slot in the lever body. The drive element may extend distally and proximally relative to the lever body. The slot may be provided between two lever body elements, potentially each of which is provided with a further through opening, such as a further slot. The further through opening or slot may extend generally perpendicular to the longitudinal axis of the instrument and/or the drive element.

The drive element may be connected to the lever by one or more contacts. The one or more contacts may be one or more drive pins or drive projections. The one or more contacts may contact the elver body, for instance a further through opening or openings provided therein.

The drive element may have a first state position and a second state position. The drive element, particularly the proximal end thereof, may be further from the casing proximal end in the first state position than in the second state position. The drive element may be within the casing in the first state position and in the second state position.

The attachment unit comprises two or more attachment elements. The attachment unit may comprise one or more connections for one or more biasing components. The one or more connections may comprise one or more pins, for instance a pin passing through an attachment element.

One or more or all of the attachment elements may be in contact with a biasing component, such as a spring, particularly a compression spring. A basing component, such as a spring, may be connected to each arm. The distal end of the spring may be connected to an arm. The proximal end of the spring may be connected to the casing, directly or indirectly. The basing component, such as a spring, may bias the attachment elements towards a second state position.

The attachment unit may have a first state position and a second state position. The attachment unit may have a disengaging state and an engaging state. The attachment unit may extend further from the casing proximal end in the disengaging state than in the engaging state.

The attachment elements have a first state position and a second state position. The attachment elements may be further apart in a first state position than the attachment elements are in the second state position, particularly the distal ends thereof. The attachment elements may be rotationally further apart in the first state position than in the second state position, particularly the distal ends thereof. The attachment elements may subtend a greater angle between them in the first state position than in the second state position. The attachment elements may, in use, contact an implant and/or a trial implant in the second state position. The attachment elements may, in use, be spaced from an implant and/or trial implant in the first state position. The attachment elements may, in use, engage with an implant and/or trial implant in the second state position.

One or more or all of the attachment elements may be arms. One or more of the arms may sub-divide, for instance to give two or more sub-arms. Optionally two attachment elements in the form of arms may be provided. Optionally two attachment elements in the form of arms, each in the form of two sub-arms may be provided. One or more or all of the attachment elements and/or arms and/or sub-arms may be provided with a distal end. The distal end[s] may be spaced from the casing. One or more or all of the attachment elements and/or arms and/or sub-arms may be provided with a proximal end. The proximal end[s] may be within the casing.

One or more or all of the attachment elements may be provided with one or more projections. One or more of the projections on one attachment element may be inclined towards one or more of the projections on another attachment element. The projections may be provided in opposing pairs. Optionally two projections may be provided on each of two attachment elements. Optionally two projections may be provided on each of two arms. Optionally one projection may be provided on each of two sub-arms. One or more or all of the projections may, in use, enter a recess in an implant or trial implant, for instance in the second position state.

One or both or more or all attachment elements are mounted on the casing. One or both or more or all attachment elements include a pivot. One or more or all of the pivots may be constrained by the casing, for instance be being mounted on the casing. One or both or more or all of the pivots may be movable relative to the casing. For instance, an attachment element pivot may have a distal position. For instance, an attachment element pivot may have a proximal position. An attachment element pivot may be slidably received in the casing, for instance a slot in the casing.

The attachment element pivots provide the pivots about which attachment elements rotate between the first state position and the second state position. The attachment element pivots provide the pivots about which attachment elements rotate between the first state position and the second state position, when at a most distal end position relative to the casing. The most distal end position may be defined by the abutment of the attachment arm pivot with the distal end of a constraint on the casing, such as the distal end of a slot in the casing.

The attachment unit is connected to the drive element by a connection. The connection may include a further pivot. The further pivot may be constrained by the casing, for instance by being mounted on the casing. The further pivot is moveable relative to the casing. For instance, a further pivot may have a distal position. For instance, a further pivot may have a proximal position. For instance, a further pivot may have an intermediate position, potentially halfway between the proximal position and the distal position. One or more further pivots may be slidably received in the casing, for instance in a further slot in the casing.

The further pivot[s] may provide the drive for the attachment elements to rotate between the first state position and the second state position. The further pivot[s] may provide distal movement of a part of the attachment elements to rotate them between the first state position and the second state position. The further pivot[s] may drive the rotation of the attachment locations when transitioning from an intermediate position to a more distal end position relative to the casing, such from a halfway position and/to their most distal end position relative to the casing. The most distal end position may be defined by the abutment of the further pivot[s] with the distal end of a constraint on the casing, such as the distal end of a further slot in the casing.

The instrument may have a disengaging state and an engaging state. One or more or all of the attachment elements may be in the first state position when the instrument is in the disengaging state. One or more or all of the attachment elements may be in the second state position when the instrument is in the engaging state. The user interface may be in a depressed state when the instrument is in the disengaging state. The user interface may be in an elevated state when the instrument is in the engaging state.

In the disengaging state, in use, a surgical component may be brought into proximity with the distal end of the instrument. At least a part of the surgical component may be provided between two or more attachment elements, in use, with the instrument in the disengaging state.

The instrument may transition from the disengaging state to the engaging state. The transition may include a first transition phase. The first transition phase may provide engagement of the attachment elements with a surgical component. The transition may include a second transition phase, for instance provided after the first transition phase. The second phase may provide engagement of the casing with a surgical component. The first transition phase and the second transition phase may be discrete form one another.

The instrument may have a first transition opening position. The first transition opening position may be the disengaging state and/or first state position. The instrument may have a second transition ending position. The second transition ending position may be the engaging state.

The instrument may have one or more intermediate positions between the first transition opening position and the second transition ending position. The one or more intermediate positions may include a first transition ending position. The one or more intermediate positions may include a second transition opening position. The first transition ending position may be the same as the second transition opening position.

The attachment elements may transition from the first state position to the second state position. This transition may include a first transition phase. The transition may include a second transition phase, with the attachment elements remaining in the second state position during second transition phase. The second transition phase may be provided after the first transition phase. The attachment element transition phases and/or their positions may correspond to the instrument transition phases and/or their positions.

In the first transition opening position, one or more of the following may be provided:
The user interface may be in a depressed state; and/or
The drive element may be in a first state position; and/or
The biasing component may be in a non-relaxed configuration, for instance a spring under compression; and/or
The attachment elements may have an increased spacing and/or a gap between them larger than a corresponding dimension of the surgical component; and/or
The attachment elements may have, at their distal ends, a maximum angular level of separation; and/or
The attachment element pivots may be in a first state position; and/or
The further pivot[s] may be on a distal side of a line projected between two attachment element pivots; and/or
The further pivot[s]may be in a first state position; and/or
The opposing projections on the instrument may have a gap between them greater than the gap between opposing recesses in a surgical component; and/or
The casing distal end and/or surgical component abutment surface may be spaced from any surgical component.

In the first transition ending position and/or second transition opening position, one or more of the following may be provided:
The user interface may be in an intermediate state between a depressed state and an elevated state; and/or
The drive element may be in an intermediate state position, proximal of the first state position and/or distal of the second state position; and/or
The biasing component may be in a partial non-relaxed configuration, for instance a spring under intermediate compression; and/or
The attachment elements may have a reduced spacing and/or a gap between them; and/or
The attachment elements may have, at their distal ends, a reduced angular level of separation; and/or
The attachment elements may be in contact with a surgical component, for instance in an engagement with a surgical component; and/or
The attachment element pivots may be in a second state position; and/or
The further pivot[s] may be in an intermediate state position, proximal of the first state position and/or distal of the second state position; and/or
The further pivot[s] may be on a line projected between two attachment element pivots; and/or
The opposing projections on the instrument may have entered opposing recesses in a surgical component; and/or
The casing distal end and/or surgical component abutment surface may be spaced from any surgical component.

In the second transition ending position, one or more of the following may be provided:
The user interface may be in an elevated state; and/or
The drive element may be in a second state position; and/or
The biasing component may be in a relaxed or more relaxed configuration, for instance a spring under low or no compression; and/or
The attachment elements may have a reduced spacing and/or a gap between them; and/or
The attachment elements may have, at their distal ends, a reduced angular level of separation; and/or
The attachment elements may be in contact with a surgical component, for instance in an engagement with a surgical component; and/or
The attachment element pivots may be in a second state position; and/or
The further pivot[s] may be in a second state position, potentially proximal of the first state position and/or proximal of the intermediate state position; and/or
The further pivot[s] may be on a proximal side of a line projected between two attachment element pivots; and/or
The opposing projections on the instrument may have entered opposing recesses in a surgical component; and/or
The casing distal end and/or surgical component abutment surface may be in abutment with a surgical component.

In the engaging state, in use, a surgical component may be held by a distal portion and/or intermediate portion of the instrument. At least a part of the surgical component may be engaged by two or more attachment elements, in use, with the instrument in the engaging state. In the engaging state, in use, the surgical component may be brought into proximity with a surgical site. In the engaging state, in use, the surgical component may be mounted on a surgical site. In the engaging state, in use, the surgical component may be impacted by the surgical instrument and/or the surgical instrument may convey impacts applied to the proximal end of the instrument to the distal end of the instrument and/or to the surgical component. In the engaging state, the surgical component may be removed from a surgical site.

The instrument may transition from the engaging state to the disengaging state. The transition may include a fourth transition phase. The fourth transition phase may provide disengagement of the attachment elements from a surgical component. The transition may include a third transition phase, for instance provided before a fourth transition phase. The third transition phase may provide removal of a surgical component from the casing. The third transition phase and the fourth transition phase may be discrete form one another. The transition may include a fourth transition phase. The fourth transition phase may provide disengagement of the attachment elements from a surgical component. The third transition phase may be the reverse of the second transition phase. The fourth transition phase may be the reverse of the first transition phase.

The instrument may have a third transition opening position. The third transition opening position may be the engaging state and/or second state position. The instrument may have a fourth transition ending position. The fourth transition ending position may be the disengaging state and/or first state position.

The instrument may have one or more intermediate positions between the third transition opening position and the fourth transition ending position. The one or more intermediate positions may include a third transition ending position. The one or more intermediate positions may include a fourth transition opening position. The third transition ending position may be the same as the fourth transition opening position.

The attachment elements may transition from the second state position to the first state position. This transition may include a fourth transition phase. The transition may include a third transition phase, with the attachment elements remaining in the second state position during third transition phase. The third transition phase may be provided before the fourth transition phase.

In the third transition starting position, one or more of the following may be provided:
The user interface may be in an elevated state; and/or
The drive element may be in a second state position; and/or
The biasing component may be in a relaxed or more relaxed configuration, for instance a spring under low or no compression; and/or
The attachment elements may have a reduced spacing and/or a gap between them; and/or
The attachment elements may have, at their distal ends, a reduced angular level of separation; and/or
The attachment elements may be in contact with a surgical component, for instance in an engagement with a surgical component; and/or
The attachment element pivots may be in a second state position; and/or
The further pivot[s] may be in a second state position, potentially proximal of the first state position and/or proximal of the intermediate state position; and/or
The further pivot[s] may be on a proximal side of a line projected between two attachment element pivots; and/or
The opposing projections on the instrument may have entered opposing recesses in a surgical component; and/or
The casing distal end and/or surgical component abutment surface may be in abutment with a surgical component.

In the third transition ending position and/or fourth transition opening position, one or more of the following may be provided:
The user interface may be in an intermediate state between a depressed state and an elevated state; and/or
The drive element may be in an intermediate state position, proximal of the first state position and/or distal of the second state position; and/or
The biasing component may be in a partial non-relaxed configuration, for instance a spring under intermediate compression; and/or
The attachment elements may have a reduced spacing and/or a gap between them; and/or
The attachment elements may have, at their distal ends, a reduced angular level of separation; and/or
The attachment elements may be in contact with a surgical component, for instance in an engagement with a surgical component; and/or
The attachment element pivots may be in a second state position; and/or
The further pivot[s] may be in an intermediate state position, proximal of the first state position and/or distal of the second state position; and/or
The further pivot[s] may be on a line projected between two attachment element pivots; and/or
The opposing projections on the instrument may have entered opposing recesses in a surgical component; and/or
The casing distal end and/or surgical component abutment surface may be spaced from any surgical component.

In the fourth transition ending position, one or more of the following may be provided:
The user interface may be in a depressed state; and/or
The drive element may be in a first state position; and/or
The biasing component may be in a non-relaxed configuration, for instance a spring under compression; and/or
The attachment elements may have an increased spacing and/or a gap between them larger than a corresponding dimension of the surgical component; and/or
The attachment elements may have, at their distal ends, a maximum angular level of separation; and/or
The attachment element pivots may be in a first state position; and/or
The further pivot[s] may be on a distal side of a line projected between two attachment element pivots; and/or
The further pivot[s]may be in a first state position; and/or
The opposing projections on the instrument may have a gap between them greater than the gap between opposing recesses in a surgical component; and/or
The casing distal end and/or surgical component abutment surface may be spaced from any surgical component.

The first aspect of the disclosure may include any of the other feature features, options or possibilities set out herein, including in the other aspects of the disclosure, and including when any are taken singularly or in any combination.

According to a second aspect the disclosure may provide a kit, the kit comprising:
one or more surgical components; and
the surgical instrument, in which the surgical instrument is adapted to engage with the surgical component.

The kit may include one or more surgical components. The surgical component may be a trial surgical component. The surgical component may be a surgical implant. The surgical component may be a trial implant. The kit may include a plurality of surgical components and/or surgical implants and/or trial surgical implants.

The kit may include one or more further surgical instruments. The one or more further surgical instruments may include an impactor or mallet.

The surgical implant may be a femoral implant, such as a unicondylar femoral implant.

The kit may include one or more surgical components for the surgical instrument to engage. The surgical component may be provided with one or more engagement locations, for instance one or more recesses. At least one recess may be provided on each side of the surgical component. At least two recesses may be provided on each side of the surgical component. The two recesses may be provided as a pair. The spacing between recesses on the same side or edge of the surgical component may be the same for different sizes and/or geometries of surgical component.

The trial surgical component may be provided with one or more engagement locations, for instance one or more recesses. At least one recess may be provided on each side of the trial surgical component. At least two recesses may be provided on each side of the trial surgical component. The two recesses may be provided as a pair. The spacing between recesses on the same side or edge of the trial surgical component may be the same for different sizes and/or geometries of trial surgical component.

The spacing between recesses on the same side or edge of the trial surgical component may be the same for different sizes and/or geometries of trial surgical component and may also be the same for different sizes and/or geometries of surgical component.

The kit may include a series of trial surgical components which are different from one another. The differences may be incremental differences. The differences may be in the dimensions of one or more parts of the trial surgical components. The differences may be in the geometry of one or more parts of the trial surgical components. The kit may include a number of medial trial surgical components and/or a number of lateral trial surgical components.

The kit may include a series of surgical components which correspond to a trial surgical component in terms of the dimensions of one or more parts of the trial surgical components and/or in terms of the geometry of one or more parts of the trial surgical components.

The second aspect of the disclosure may include any of the other features, options or possibilities set out herein, including in the other aspects of the disclosure, and including when any are taken singularly or in any combination.

According to further information useful as background only there is provided a method of providing a surgical component at a surgical site, the method comprising:
providing one or more surgical components;
providing a surgical instrument for engagement with at least one of the surgical components, the surgical instrument including an attachment unit, the attachment unit including one or more attachment elements, one or more of the attachment elements being rotatably mounted, the attachment elements being in a first state position;
engaging the surgical component with the surgical instrument by transitioning the attachment elements from the first state position to a second state position in which the attachment elements engage with the at least one surgical component; and
introducing the surgical component to the surgical site using the surgical instrument with the attachment elements in the second state position.

The method may include the instrument having an engaging state. One or more or all of the attachment elements may be in the second state position when the instrument is in the engaging state. The user interface may be in an elevated state when the instrument is in the engaging state. The surgical component may be introduced to the surgical site with the instrument in the engaging state.

The method may include introducing the surgical component to the surgical site using the surgical instrument in the engaging state.

The method may include applying force to the instrument with the surgical component at the surgical site, for instance one or more impact force event, potentially applied to the proximal end of the surgical instrument.

Then method may include the instrument having a disengaging state. One or more or all of the attachment elements may be provided in a first state position when the instrument is in the disengaging state. The user interface may be in a depressed state when the instrument is in the disengaging state. The surgical component may be left at the surgical site by putting the instrument in the disengaging state.

The method may include leaving the surgical component at the surgical site by placing the surgical instrument in the disengaging state. The method may include leaving the surgical component at the surgical site by separating the surgical instrument from the surgical component at the surgical site.

The method may include the instrument transitioning from the engaging state to the disengaging state, for instance to provide disengagement of the attachment elements from the surgical component.

The method may include reengaging the surgical instrument with the surgical component, for instance to remove the surgical component from the surgical site.

According to further information useful as background only there is provided a method of removing a surgical component from a surgical site, the method comprising:
introducing a surgical instrument to the surgical site, the surgical instrument including an attachment unit, the attachment unit including one or more attachment elements, one or more of the attachment elements being rotatably mounted, the attachment elements being in a first state position;
the surgical site including a surgical component;
engaging the surgical component with the surgical instrument by transitioning the attachment elements from the first state position to a second state position in which the attachment elements engage with the at least one surgical component; and
removing the surgical instrument with the surgical component attached from the surgical site, with the attachment elements in the second state position.

### BRIEF DESCRIPTION OF THE DRAWINGS

Various embodiments of the disclosure will now be described, by way of example only, and with reference to the accompanying drawings in which:
Figure 1a is a perspective view of a prepared knee joint together with a prior art femoral introducer for presenting a unicondylar femoral implant to the joint;
Figure 1b is a perspective view showing the prior art femoral introducer presenting the implant to the joint;
Figure 2 is a perspective view of a first embodiment of an instrument according to the disclosure;
Figure 3 is a cross-sectional perspective view of a second embodiment of an instrument according to the disclosure;
Figure 4a is a side view of the internal mechanism of the first embodiment in an engaging state;
Figure 4b is a plan view of the internal mechanism of Figure 4a;
Figure 5a is a side view of the internal mechanism of the first embodiment in a disengaging state;
Figure 6 is a plan view of a detail of the first embodiment in a disengaging state;

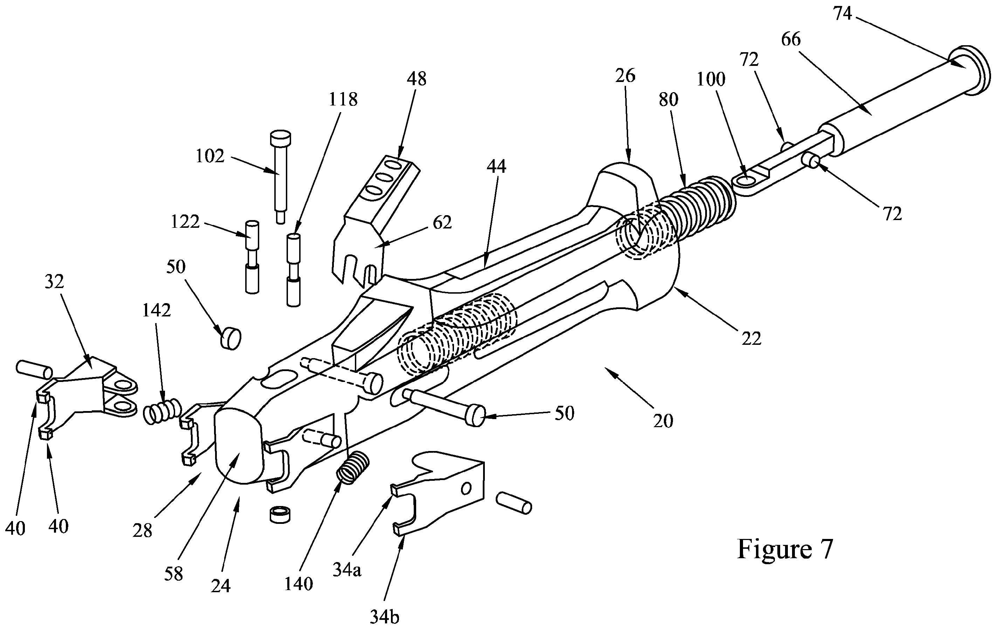

Figure 7 shows a perspective view of an embodiment in both assembled and exploded views;
Figure 8 shows a tibial trial and femoral trial combination in a surgical site;
Figure 9 is a perspective view of an implant showing the recesses therein that the instrument engages with; and
Figure 10 shows the states and transitions for an embodiment of an instrument, in use.

### DETAILED DESCRIPTION OF THE DRAWINGS

In partial or total knee arthroplasty, one or more of the load bearing surfaces in the knee joint are replaced. Typically, this involves removing the natural load bearing surface[s] or previously replaced load bearing surface[s] and replacing them with new implanted load bearing surface[s]. The femoral and tibial bearing surfaces are typically replaced.

In the case of the femoral implant, once the joint has been prepared to receive the implant, a trial implant is introduced and the movement tested to establish the most appropriate size and geometry for the final implant choice. Typically, a number of trials need to be introduced, tested and then removed one by one. Once the necessary verification of the correct size and/or geometry has been confirmed using the trials, then the final implant is introduced.

Introduction and removal of the trials and the implant is usually achieved using a femoral component introducer.

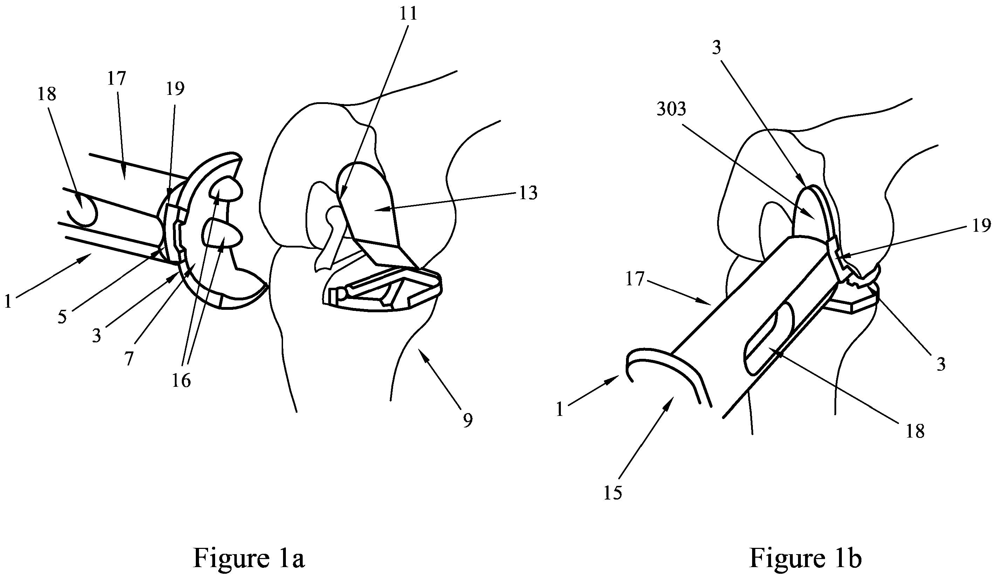

Figure 1a and 1b illustrate a prior art femoral introducer 1, in use to introduce a unicondylar femoral implant 3. The femoral implant 3 is mounted on the distal end 5 of the femoral introducer 1 remote from the surgical site and a layer of cement 7 is applied. The femoral introducer 1 is then brought into proximity with the surgical site 9 as shown in Figure 1a. The cut bone 11 has had cement 13 applied, including into the lug holes [hidden by the cement 13]. The femoral introducer 1 is used to manipulate the femoral implant 3 into position, Figure 1b, with the knee flexed. A mallet [not shown] is used to impact the proximal end 15 of the femoral introducer 1 to tap the lugs 16 into the lug holes and generally seat the femoral implant 3 on the cut bone 11. The femoral introducer 1 is then released to allow its removal leaving the femoral implant 3 in-situ.

The same femoral introducer 1 is used for the introduction and removal of trials.

The femoral introducer 1 consists of a casing 17 which can be slid relative to two pieces of sprung steel 18. The distal ends of the sprung steel 18 are spaced apart and each include inward facing projections 19. With the casing 17 retracted, the two pieces of sprung steel 18 open further apart and the projections 10 can be positioned around and adjacent the femoral implant 3. The casing 17 is then slid distally which causes the two pieces of sprung steel 18 to be forced inward towards each other and so bring the projections 19 into a firm engage with the femoral implant 3. To release the femoral implant 3, it is necessary to retract the casing 17 and this allows the two pieces of sprung steel to open outwardly.

For successful joint reconstruction, it is necessary to offer the surgeon the choice of a plurality of different sizes of implant and potentially different geometries. For instance, the medial condyle and the lateral condyle will have different geometries. Typically six different sizes may be provided, together with different medial and lateral geometries; twelve different sizes/geometries. To enable testing, an equivalent range of sizes and geometries of trials is also provided.

The disclosure seeks to provide an instrument, such as a femoral introducer, which provides a more robust connection between the instrument and the implant and/or trials across the full range of sizes and geometries necessary. As well as seeking to provide improved robustness and stability of connection, the instrument may be capable of being quickly and efficiently loaded with an implant and/or trials and also unloaded so as to avoid negative impacts upon workflow and efficiency during surgery.

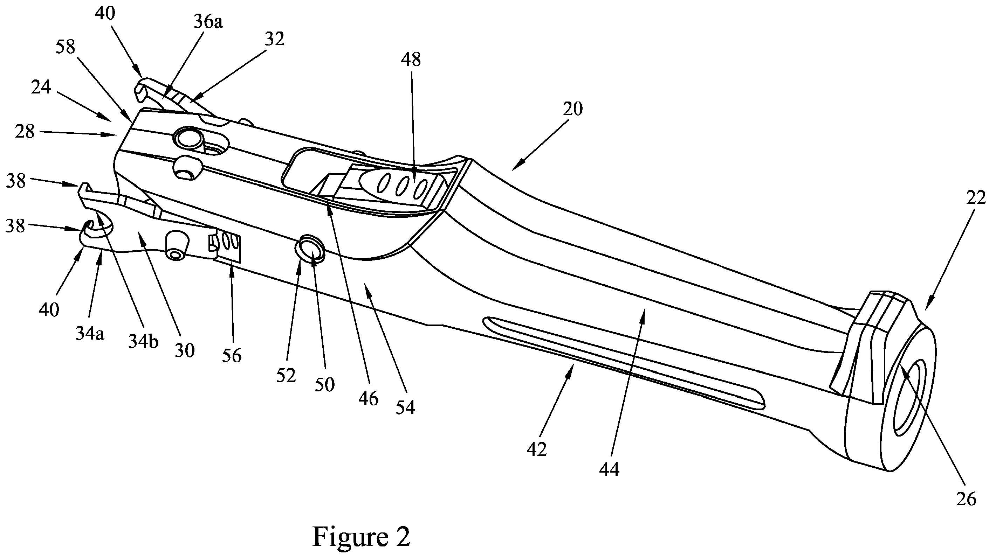

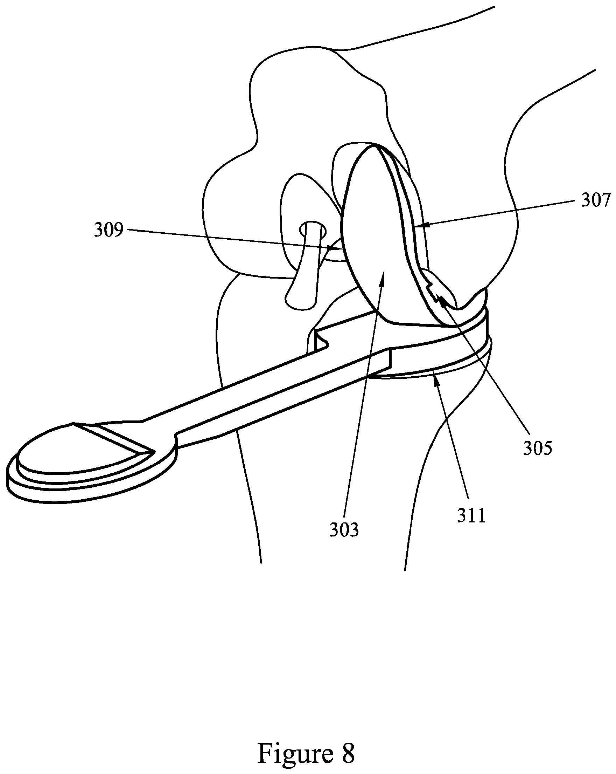

Figure 2 shows a perspective view of an instrument, in this case a femoral introducer 20, which has a proximal end 22 and a distal end 24. The proximal end 22 provides a flat surface 26 to which impacts can be applied, in use, to apply force to the implant and/or trial. The distal end 24 provides a mounting location 28 for the implant 3 and/or trial 303 shown in Figure 8.

Figure 2 shows the mounting location 28, as an example of an attachment unit, in a disengaging state with an increased separation between the opposing pair of arms formed by arm 30 and arm 32; an example of a first state position and of attachment elements. Each arm 30, 32 is divided into two sub-arms 34a, 34b and 36a and 36b [with 36b obscured in Fig 2]. At the distal end 38 of each of the sub-arms 34a, 34b, 36a and 36b there is a projection 40. The projections 40 on one set of sub arms 34a, 24b generally face the projections 40 on the opposing set of sub-arms 36a, 36b.

Figure 2 shows a casing 42 for the mechanism of the femoral introducer 20, including a body section 44 which is profiled for easy grip by the surgeon. Distal of this body section 44 is an opening 46 which provides user access to a lever 48, as an example user interface.

As shown in Figure 2, the lever 48 is in a depressed state to affect the releasing state for the mounting location 28. The lever 48 is pivotally mounted on a shaft 50 rotatably mounted in apertures 52 in the side wall 54 of the casing 42.

At the distal end 54 of the casing 42 a slot 56 is provided in the side wall 54 on each side so as to allow movement of the arms 30, 32 between the releasing state and the engaging state. The distal end 54 also provides a contact surface 58 which, in use, abuts an anterior surface of the implant 3 or trial 303 and provides for the transfer of moderate impacts to the implant 3 or trial 303 to seat them on the femur.

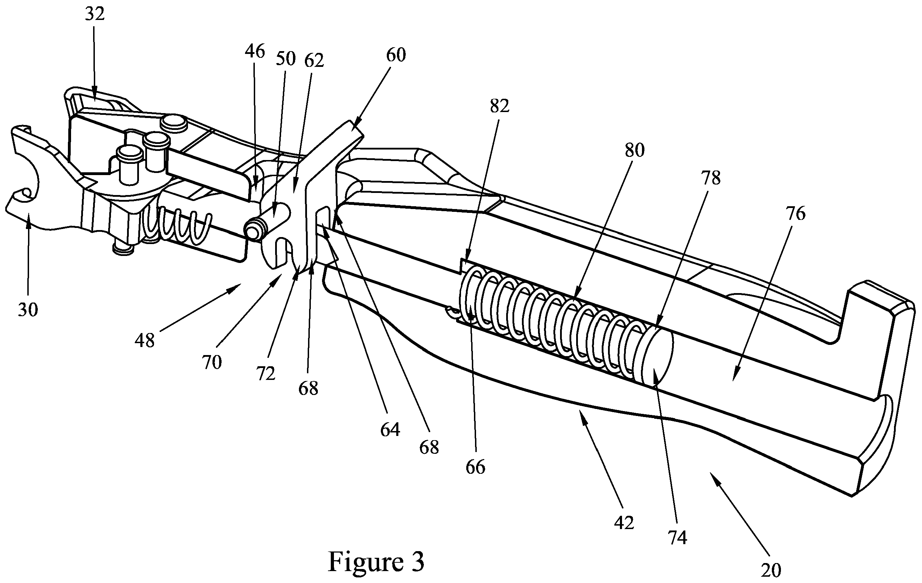

Figure 3 shows a closely related but different embodiment [primarily in terms of the casing profile] of the femoral introducer 20 in the engaging state with the reduced separation between the opposing pair of arms formed by arms 30, 32; and example of a second state position.

In the engaging state, the lever 48 is in an elevated state and the user interface part 60 protrudes from the opening 46. The user interface part 60 provides a convenient flat, but ribbed surface for cooperation with the surgeon's thumb. The user interface part is connected to a lever body 62 from which the shaft 50 or shafts 50 project transverse to the casing 42.

The lever body 62 includes a through opening, a slot 64 in the illustrated embodiment, which allows a drive element 66 to extend distally and proximally relative to the lever body 62. The slot 64 is provided between two lever body elements 68 each of which is provided with a further through opening, again a further slot 70 in the illustrated embodiment. The further slots 70 extend generally perpendicular to the slot 64 and/or to the longitudinal axis of the femoral introducer 20 and/or the drive element 66. The drive element 66 is provided with a pair of drive projections 72, with one engaging with each of the further slots 70.

As a result of this configuration, if the lever 48 is moved from the elevated state of Figure 3 towards the depressed state of Figure 2, then the lever body 62 rotates proximally about the fixed shaft(s) 50 and the lever body elements 68 are also rotated together with the further slots 70 which rotate distally. The further slots 70 transfer that movement to the drive projections 72 and hence to the drive element 66. The drive element 66 is advanced distally as a result.

The drive element proximal end 74 is received in a cavity 76, such as a bore, in the casing 42. The drive element proximal end 74 is provided with a retaining flange 78 for one end of a compression spring 80, as an example biasing component, and the other end of the spring 80 abuts the bore distal end 82. As a result, movement of the lever 48 from the elevated state towards the depressed state compresses the spring 80. If the lever 48 is released then the spring 80 urges the drive element 66 and connected parts to return the lever 48 to the elevated state.

As described in more detail below, the transition from the elevated state to the depressed state for the lever 48 also causes the transition of the mounting location 28 from an engaging state to a releasing state. Equally, the spring 80 urges the mounting location towards an engaging state when the lever 48 is eased or released. Thus the spring 80 seeks to maintain the introducer 20 in the engaging state and the level 48 in the elevated state.

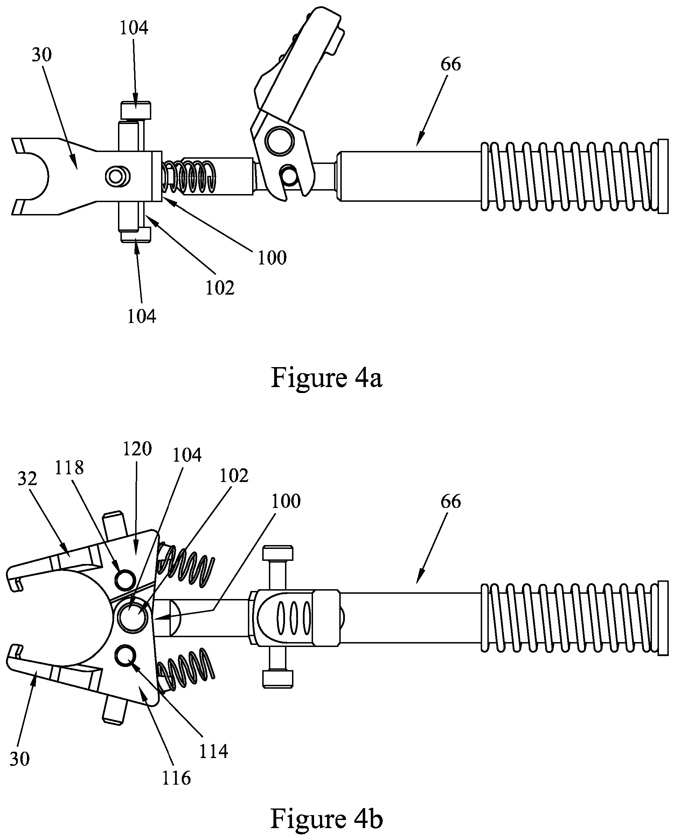

Figure 4a shows the embodiment of Figure 2 in the elevated state for the lever 48 and engaging state for the mounting location 28, in side view. Figure 4b shows the embodiment of Figure 2 and Figure 4a in plan view.

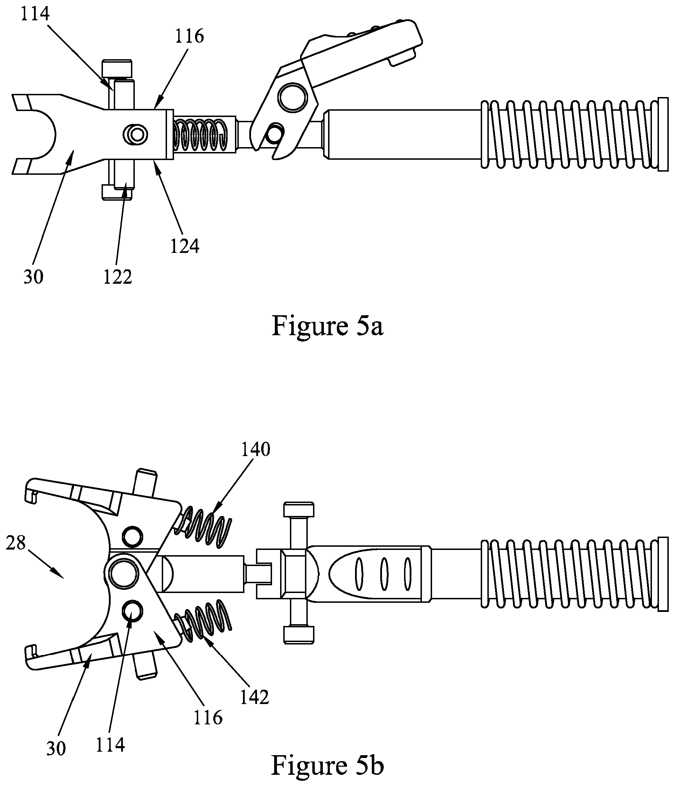

Figure 5a shows the embodiment of Figure 2 in the depressed state for the lever 48 and releasing state for the mounting location 28, again in a side view. Figure 5b shows the embodiment of Figure 2 and Figure 5a in plan view.

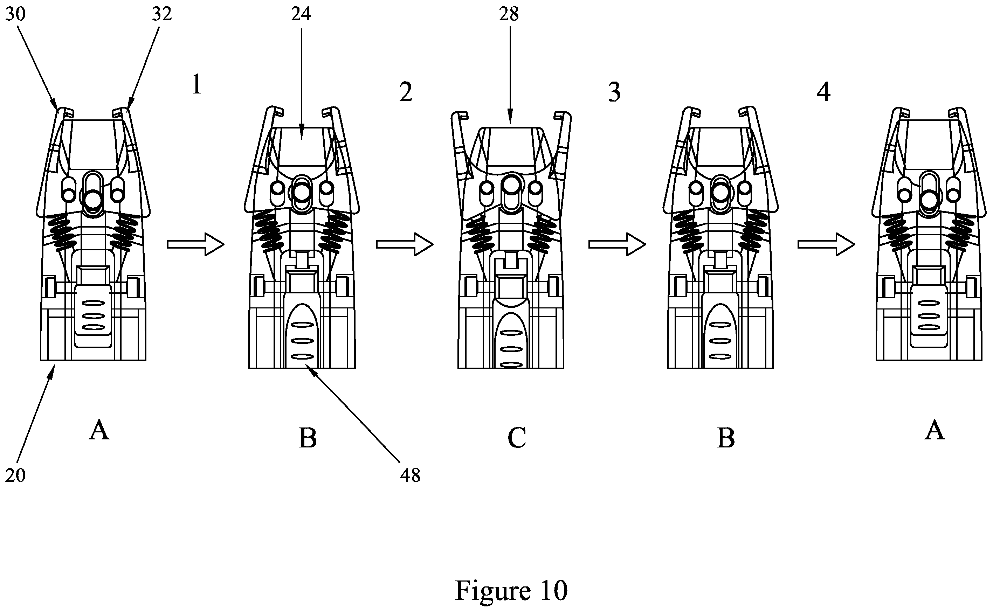

Before describing the operation of the femoral inducer 20 in detail, it is useful to consider the states and the sequence of transitions for the femoral introducer 20 shown in Figure 10. Working from left to right, Figure 10 shows:
a. Configuration A - the femoral introducer 20 in the engaging state, with the arms 30, 32 [attachment elements] in the second state position, the lever 48 [user interface] in the elevated state and the mounting location 28 [attachment unit] in the engaging state;
b. Arrow 1 - the second transition phase which moves the surgical component out of abutment with the distal end 24 of the femoral introducer 20, but still firmly retains the surgical component mounted on the femoral introducer 20;
c. Configuration B - the femoral introducer 20 in the engaging state, with the arms 30, 32 [attachment elements] in the second state position, the lever 48 [user interface] in an intermediate state and the mounting location 28 [attachment unit] in the engaging state;
d. Arrow 2 - the first transition phase which increases the separation between the opposing arms 30, 32 and so disengages the surgical component from being mounted on the femoral introducer 20;
e. Configuration C - the femoral introducer 20 in the disengaging state, with the arms 30, 32 [attachment elements] in the first state position, the lever 48 [user interface] in a depressed state and the mounting location 28 [attachment unit] in the disengaging state;
f. Arrow 3 - reversal of the first transition phase;
g. Configuration B provided again;
h. Arrow 4 - reversal of the second transition phase;
i. Configuration A provided again.

Returning to Figures 4a and 4b, together with Figures 5a and 5b, although the opposing arms 30, 32 are pivotally mounted, the movement is more complex than a simple rotation of the arms to open them by varying their spacing. A first transition phase occurs and then a second transition phase occurs.

The drive element distal end 100 is provided with an arm drive pivot 102; an example of a further pivot. As described in more detail below, movement of the arm drive pivot 102 can cause rotation of the arm 30 and arm 34. Rotation of the arm 30 and arm 32 provides the transition between the engaging state and the releasing state at the mounting location 28.

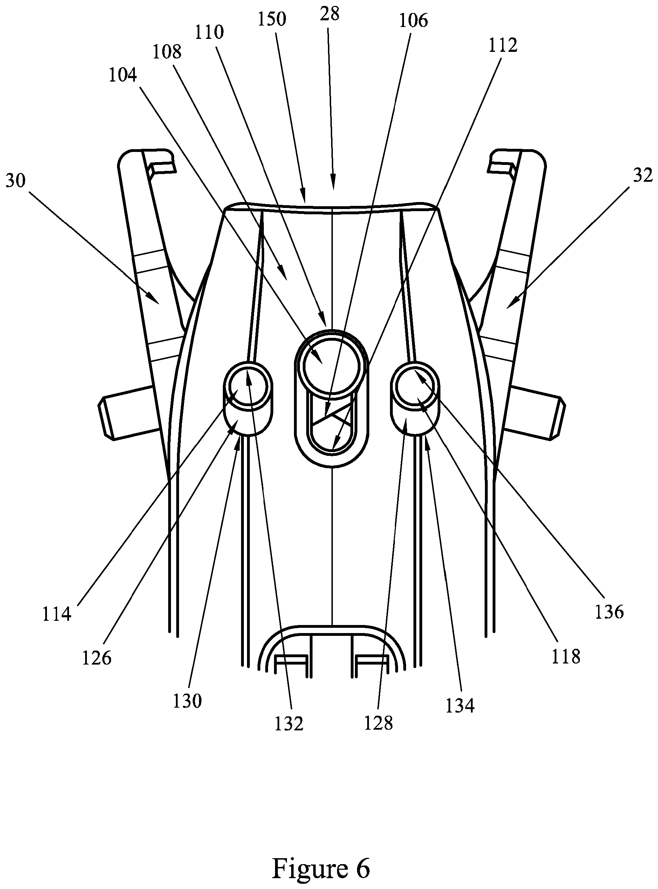

Each arm drive pivot end 104 provided on the arm drive pivot 102 is received in a drive pivot slot 106, respectively in the top wall 108 and bottom wall of the casing 42, as seen in Fig 6. The drive pivot slot 106 is configured so as to allow longitudinal movement of the pivot arm ends 104 and hence of the arm drive pivot 102 as the drive element 66 moves longitudinally.

In the elevated state for the lever 48, the engaging state for the mounting location 28, the drive element 66 is positioned more proximally within the bore 76 and so the pivot arm ends 104 are at the drive pivot slot proximal end 112 in the drive pivot slot 106.

As shown in Fig 6, in the depressed state for the lever, the disengaging state for the mounting location 28, the pivot arm ends 104 are at the drive pivot slot distal end 110 in the drive pivot slot 106. This is because as the drive element 66 is advanced distally, it carries the arm drive pivot 102 distally with it.

During the initial phase of the transition from the engaging state towards the disengaging state for the mounting location [thus prior to the position in Figure 6], as the drive element 66 advances distally, the arms 30 and 32 also advance distally and do not open. This is due to the action of an arm pivot projection 114, an example attachment element pivot, on the top surface 116 of the arm 30 and an arm pivot projection 118 on the top surface 120 of the arm 32. A similar arm pivot projection 122 is provided on the bottom surface 124 of the arm 30 and a similar one on arm 32.

The arm pivot projection 114 cooperates with pivot restraint slot 126 in the top wall 108 of the casing 42. The pivot restraint slot 126 initially allows longitudinal movement of the arm pivot projection 114 from the proximal end 130 of the pivot restrain slot 126 towards the distal end 132. Hence, as arm drive pivot 102 advances distally, the arm pivot projection 114 and the arm 30 advance too and so there is no rotation of the arm 30.

There is an equivalent manner of movement for the other arm pivot projections 118, 122. Thus arm pivot projection 118 cooperates with pivot restraint slot 128 in the top wall 108 of the casing 42. The pivot restraint slot 128 initially allows longitudinal movement of the arm restrain projection 118 from the proximal end 134 of the restrain slot 128 towards the distal end 134. Again, as arm drive pivot 102 advances distally, the arm pivot projection 118 and the arm 32 advance too and so there is no rotation of the arm 32. This can be thought of as part of the initial transition phase.

The arm pivot projections on the bottom surface 124 of the arms 30 and 34 also interact in an equivalent manner with restrain slots provided in the bottom of the casing 42 during an initial part of the transition from the engaging state towards the disengaging state.

Continued movement of the drive element 66, causes the arm pivot projections 114, 118 to abut the distal ends 132, 134 of the pivot restraint slots 126, 128 and so prevent further distal movement of the arm pivot projections 114, 118. This can be considered an initial transition phase closing position. However, the arm drive pivot 102 is still short of the distal end 110 of drive pivot slot 106 and so further movement of the drive element 66 and the arm drive pivot 102 is possible until the configuration shown in Figure 6 is reached.

In a further transition phase, as the arm drive pivot 102 continues its longitudinal movement in a distal direction, the arm drive pivot 102 moves from a position where the arm drive pivot 102 is on the proximal side of a line projected between the arm pivot projections 114, 116 [Figure 4a], to a position where the arm drive pivot 102 is on the distal side of the line projected between the arm pivot projections 114, 116 [Figure 5a]. This causes rotation of the arm 30 about the arm pivot projection 114 [anticlockwise as seen in Figure 4b and 5b] and causes rotation of the arm 32 about the arm pivot projection 116 [clockwise as seen in Figure 4b and 5b]. This causes the gap between the projections 40 on one set of sub arms 34a, 34b and the projections 40 on the opposing set of sub-arms 36a, 36b to materially increase. Eventually, the arm drive pivot end 104 reaches the drive pivot slot distal end 110 and so further movement of the arm drive pivot 102 is prevented and further rotation of the arms 30, 32 is prevented. This can be considered the further transition phase closing position.

The force conveyed from the lever 48 to the drive element 66 and to the arms 30, 32 is sufficient to overcome the return force exerted on the arms 30, 32 towards the engaging state by compression springs 140, 142, and place the mounting location 28 in the disengaging state. The distal ends of the springs 140, 142 are connected to the arms 30, 32 and the proximal ends of the springs are connected to the casing 42.

In this disengaging state of Figure 5b and 6, the femoral introducer 20 can be brought into proximity with the desired implant 3 or trial 303 to be attached.

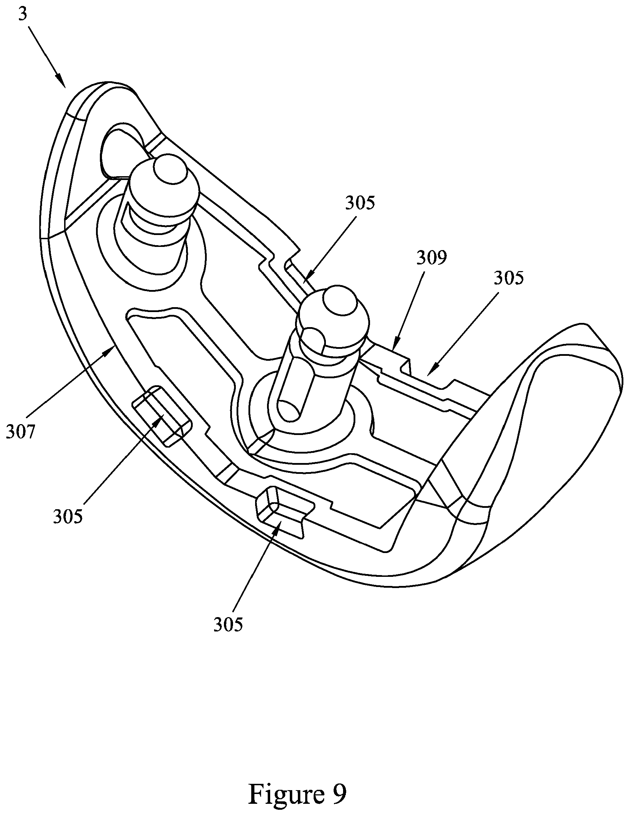

As can be seen in Figure 9, the implant 3 or trial 303 has a pair of recesses 305 on each side edge 307, 309. The recesses 305 on a side edge 307 have the same spacing along the edge 307 in all different sizes and geometries of implant 3 or trial 303. The same is true on edge 309. This spacing is the same spacing as between the two projections 40 on one set of sub arms 34a, 34b and similarly between the projections 40 on the opposing set of sub-arms 36a, 36b.

With the implant 3 or trial 303 presented to the introducer 20, as the pressure on the lever 48 is eased, the compression spring 80 acting on the drive element 66 causes the drive element 66 to move proximally and this in turn brings the pair of projections on the sub-arms 34a, 34b and the pair of projections 40 on the sub-arm 36a, 36b towards each other and into engagement with the recesses 305 on the implant 3 or trial 303. This action occurs because, as the arm drive pivot 102 starts its longitudinal movement in a proximal distal direction, the arm drive pivot 102 moves from a position where the arm drive pivot 102 is on the distal side of a line projected between the arm pivot projections 114, 116 [Figure 5a], to a position where the arm drive pivot 102 is on the proximal side of the line projected between the arm pivot projections 114, 116 [Figure 4a]. This allows the compression spring 140 to cause rotation of the arm 30 about the arm pivot projection 114 [clockwise as seen in Figure 4b and 5b] and allows the compression spring 142 to cause rotation of the arm 32 about the arm pivot projection 116 [anticlockwise as seen in Figure 4b and 5b]. This causes the gap between the projections 40 on one set of sub arms 34a, 34b and the projections 40 on the opposing set of sub-arms 36a, 36b to materially decrease. The projections 40 engage the recesses 305 firmly.

With the projections 40 engaged with the recesses 305 on the implant 3 or trial 303, further easing of the lever 48 allows further proximal movement of the drive element 66, arm drive pivot 102 and of the arms 30, 32. This brings the implant 3 or trial 303 closer to the introducer 20.

Hence, the release of the lever 48 causes first closure of the arms 30, 32 in a first movement phase and then retraction of the arms 30, 32 towards the casing 42 in a second movement phase. The second movement phase brings the implant 3 or trial 303 towards and then into abutment with the distal end 24 of the introducer 20. The distal end 24 is defined by a contact face 150 which is profiled to provide a significant contact area with the implant 3 or trial 303. In this way, the impact force, when applied, is transferred to the implant 3 or trial 303 by the contact face 150 without detriment to the bearing surface 311 of the implant 3 [marked in Figure 1b for guidance] or the bearing surface 311 of the trial 303 [shown in Figure 8] that it is in contact with.

The disclosure seeks to provide improved stability for the implant 3 and/or trial 303 on the introducer 20. For the full-size range of implants 3 and trials 303 that the introducer 20 is to be used with. The manner in which the engaging state is provided means that there is less flex and toggle for the femoral implant 3 and/or trial 303 when mounted on the introducer 20. The introducer 20 is also more accommodating on loose tolerancing on the implant holding geometry whilst still giving a stable mount. This is due to the two phase movement, as the instrument is able to strongly engage the implant and/or trial at each of a wider range of separations of the arms 30, 32 and the projections 40 provided thereon. Improved stability in turn gives better positioning for the implant 3 and/or trial 303 in the surgical site 9.

The manner in which the introducer 20 is able to open to a disengaging state also means that less force needs to be applied by the surgeon and the introducer 20 is generally more comfortable to use and operate. Ergonomically, the user interface is easier to reach and operate. In addition, the two phase movement delivers sufficient holding and retaining force for the implant and/or trial on the instrument, whilst only requiring a relatively low level of force from the user to causes the movement phases.

While the concepts of the present disclosure are susceptible to various modifications and alternative forms, specific embodiments thereof have been shown by way of example in the drawings and will be described herein in detail. It should be understood, however, that there is no intent to limit the concepts of the present disclosure to the particular forms disclosed, but on the contrary, the intention is to cover all modifications and alternatives that fall within the scope of the invention, which is defined by the appended claims.

## Claims

1. A surgical instrument (20), the instrument (20) comprising:
a casing (42), the casing (42) having a casing proximal end (22) and a casing distal end (24);
a drive element (66), the drive element (66) being slidably mounted within the casing (42), the drive element (66) having a drive element distal end (100);
an attachment unit connected to the drive element distal end (100), the attachment unit including two or more attachment elements (30, 32), the attachment elements (30, 32) having a first state position and a second state position;
wherein one or more of the attachment elements (30, 32) are rotatably mounted;
wherein an attachment element (30, 32) is mounted on the casing (42) by an attachment element pivot (114) and the attachment element pivot (114) is movable relative to the casing (42);
wherein the attachment unit is connected to the drive element (66) by a connection and the connection is a further pivot (102), wherein the further pivot (102) is moveable relative to the casing (42);
characterised wherein the attachment element pivot (114) has a distal position and a proximal position, the attachment element pivot (114) being slidably received in the casing (42) and/or wherein the attachment element pivot (114) provides the pivot (114) about which an attachment element (30, 32) rotates between the first state position and the second state position when the attachment element pivot (114) is at a distal end position relative to the casing (42).

2. An instrument (20) according to claim 1, wherein the attachment elements (30,32) do not rotate about attachment element pivots (114) when at a more proximal position relative to the casing (42).

3. An instrument (20) according to claim 1 or 2, wherein the further pivot (102) has a distal position and a proximal position and an intermediate position, the further pivot (102) being slidably received in the casing (42) and wherein the further pivot (102) drives the rotation of the attachment elements (30, 32) when transitioning from the intermediate position to the distal position relative to the casing.

4. An instrument (20) according to claim 3, wherein the distal position is defined by the abutment of the further pivot (102) with a distal end of a constraint on the casing (42).

5. An instrument (20) according to any preceding claim, wherein the attachment elements (30, 32) transition from the first state position to the second state position, the transition includes a first transition phase and the transition includes a second transition phase, with the attachment elements (30, 32) remaining in the second state position during second transition phase.

6. An instrument (20) according to any preceding claim, wherein the distal ends (38) of the attachment elements (30, 32) are rotationally further apart in the first state position than in the second state position.

7. An instrument (20) according to any preceding claim, wherein one or more of the attachment elements (30, 32) are arms (30, 32) and one or more of the arms (30, 32) sub-divide to give two or more sub-arms (34a, 34b, 36a, 36b) and/or wherein the attachment elements (30, 32) are provided with one or more projections (40) at or towards their distal ends (38), the one or more projections (40) on one attachment element (30, 32) being inclined towards one or more of the projections (40) on another attachment element (30, 32) and/or wherein one or more of the attachment elements (30, 32) are connected to a biasing component (80) and the basing component (80) biases the attachment elements (30, 32) towards the second state position.

8. An instrument (20) according to any preceding claim, wherein the casing proximal end (22) provides an impact surface (150) and/or wherein the casing distal end (24) provides an abutment surface for a surgical component.

9. An instrument (20) according to any preceding claim, wherein a user interface (48) is operably connected to the drive element (66) and rotation of the user interface (48) causes longitudinal movement of the drive element (66) and the attachment unit.

10. An instrument (20) according to any preceding claim, wherein the drive unit (48) is biased towards the proximal end (22) of the casing (42) or is biased towards the distal end (24) of the casing (42) and/or wherein the drive element (66) has a first state position and a second state position and the proximal end of the drive element (66) is further from the casing proximal end (22) in the first state position than in the second state position.

11. A kit, the kit comprising:
the surgical instrument (20) according to claim 1; and
one or more surgical components with which the surgical instrument (20) engages, the surgical component comprising one or more engagement locations;
wherein the surgical instrument (20) is adapted to engage with the surgical component.

12. A kit according to claim 11, wherein the kit includes an impactor and/or mallet and/or wherein the kit includes a series of trial surgical components (303) which are different from one another in the dimensions of one or more parts of the trial surgical components (303) and/or in the geometry of one or more parts of the trial surgical components (303).

## Patentansprüche

1. Ein chirurgisches Instrument (20), wobei das Instrument (20) Folgendes beinhaltet:
ein Gehäuse (42), wobei das Gehäuse (42) ein proximales Gehäuseende (22) und ein distales Gehäuseende (24) aufweist;
ein Antriebselement (66), wobei das Antriebselement (66) verschiebbar innerhalb des Gehäuses (42) montiert ist, wobei das Antriebselement (66) ein distales Antriebselementende (100) aufweist;
eine Befestigungseinheit, die mit dem distalen Antriebselementende (100) verbunden ist, wobei die Befestigungseinheit zwei oder mehr Befestigungselemente (30, 32) umfasst, wobei die Befestigungselemente (30, 32) eine erste Zustandsposition und eine zweite Zustandsposition aufweisen;
wobei eines oder mehrere der Befestigungselemente (30, 32) drehbar montiert sind;
wobei ein Befestigungselement (30, 32) durch einen Befestigungselementdrehzapfen (114) an dem Gehäuse (42) montiert ist und der Befestigungselementdrehzapfen (114) relativ zu dem Gehäuse (42) beweglich ist; wobei die Befestigungseinheit durch eine Verbindung mit dem Antriebselement (66) verbunden ist und die Verbindung ein weiterer Drehzapfen (102) ist, wobei der weitere Drehzapfen (102) relativ zu dem Gehäuse (42) beweglich ist;
gekennzeichnet wobei der Befestigungselementdrehzapfen (114) eine distale Position und eine proximale Position aufweist, wobei der Befestigungselementdrehzapfen (114) verschiebbar in dem Gehäuse (42) aufgenommen ist und/oder wobei der Befestigungselementdrehzapfen (114) den Drehzapfen (114) bereitstellt, um den sich ein Befestigungselement (30, 32) zwischen der ersten Zustandsposition und der zweiten Zustandsposition dreht, wenn sich der Befestigungselementdrehzapfen (114) relativ zu dem Gehäuse (42) an einer distalen Endposition befindet.

2. Instrument (20) gemäß Anspruch 1, wobei sich die Befestigungselemente (30, 32) nicht um Befestigungselementdrehzapfen (114) drehen, wenn sie sich an einer proximaleren Position relativ zu dem Gehäuse (42) befinden.

3. Instrument (20) gemäß Anspruch 1 oder 2, wobei der weitere Drehzapfen (102) eine distale Position und eine proximale Position und eine Zwischenposition aufweist, wobei der weitere Drehzapfen (102) verschiebbar in dem Gehäuse (42) aufgenommen ist und wobei der weitere Drehzapfen (102) die Drehung der Befestigungselemente (30, 32) antreibt, wenn er relativ zu dem Gehäuse von der Zwischenposition zu der distalen Position übergeht.

4. Instrument (20) gemäß Anspruch 3, wobei die distale Position durch das Anliegen des weiteren Drehzapfens (102) an einem distalen Ende einer Begrenzung auf dem Gehäuse (42) definiert ist.

5. Instrument (20) gemäß einem vorhergehenden Anspruch, wobei die Befestigungselemente (30, 32) von der ersten Zustandsposition zu der zweiten Zustandsposition übergehen, wobei der Übergang eine erste Übergangsphase umfasst und der Übergang eine zweite Übergangsphase umfasst, wobei die Befestigungselemente (30, 32) während der zweiten Übergangsphase in der zweiten Zustandsposition bleiben.

6. Instrument (20) gemäß einem vorhergehenden Anspruch, wobei die distalen Enden (38) der Befestigungselemente (30, 32) in der ersten Zustandsposition drehbezogen weiter voneinander entfernt sind als in der zweiten Zustandsposition.

7. Instrument (20) gemäß einem vorhergehenden Anspruch, wobei eines oder mehrere der Befestigungselemente (30, 32) Arme (30, 32) sind und einer oder mehrere der Arme (30, 32) unterteilt sind, um zwei oder mehr Teilarme (34a, 34b, 36a, 36b) zu ergeben, und/oder wobei die Befestigungselemente (30, 32) mit einem oder mehreren Vorsprüngen (40) an oder in Richtung ihrer distalen Enden (38) versehen sind, wobei der eine oder die mehreren Vorsprünge (40) an einem Befestigungselement (30, 32) in Richtung eines oder mehrerer der Vorsprünge (40) an einem anderen Befestigungselement (30, 32) geneigt sind, und/oder wobei eines oder mehrere der Befestigungselemente (30, 32) mit einer Vorspannkomponente (80) verbunden sind und die Vorspannkomponente (80) die Befestigungselemente (30, 32) in Richtung der zweiten Zustandsposition vorspannt.

8. Instrument (20) gemäß einem vorhergehenden Anspruch, wobei das proximale Gehäuseende (22) eine Schlagoberfläche (150) bereitstellt und/oder wobei das distale Gehäuseende (24) eine Anlageoberfläche für eine chirurgische Komponente bereitstellt.

9. Instrument (20) gemäß einem vorhergehenden Anspruch, wobei eine Benutzerschnittstelle (48) betriebsfähig mit dem Antriebselement (66) verbunden ist und eine Drehung der Benutzerschnittstelle (48) eine Längsbewegung des Antriebselements (66) und der Befestigungseinheit bewirkt.

10. Instrument (20) gemäß einem vorhergehenden Anspruch, wobei die Antriebseinheit (48) in Richtung des proximalen Endes (22) des Gehäuses (42) vorgespannt ist oder in Richtung des distalen Endes (24) des Gehäuses (42) vorgespannt ist und/oder wobei das Antriebselement (66) eine erste Zustandsposition und eine zweite Zustandsposition aufweist und das proximale Ende des Antriebselements (66) in der ersten Zustandsposition weiter von dem proximalen Gehäuseende (22) entfernt ist als in der zweiten Zustandsposition.

11. Ein Kit, wobei das Kit Folgendes beinhaltet:
das chirurgische Instrument (20) gemäß Anspruch 1; und
eine oder mehrere chirurgische Komponenten, mit denen das chirurgische Instrument (20) in Eingriff kommt, wobei die chirurgische Komponente eine oder mehrere Eingriffsstellen beinhaltet;
wobei das chirurgische Instrument (20) angepasst ist, um mit der chirurgischen Komponente in Eingriff zu kommen.

12. Kit gemäß Anspruch 11, wobei das Kit ein Schlaggerät und/oder einen Hammer umfasst und/oder wobei das Kit eine Reihe von chirurgischen Probekomponenten (303) umfasst, die sich in den Abmessungen eines oder mehrerer Teile der chirurgischen Probekomponenten (303) und/oder in der Geometrie eines oder mehrerer Teile der chirurgischen Probekomponenten (303) voneinander unterscheiden.

## Revendications

1. Un instrument chirurgical (20), l'instrument (20) comprenant :
un boîtier (42), le boîtier (42) ayant une extrémité proximale de boîtier (22) et une extrémité distale de boîtier (24) ;
un élément d'entraînement (66), l'élément d'entraînement (66) étant monté de manière coulissante à l'intérieur du boîtier (42), l'élément d'entraînement (66) ayant une extrémité distale d'élément d'entraînement (100) ;
une unité de fixation raccordée à l'extrémité distale d'élément d'entraînement (100), l'unité de fixation incluant deux éléments de fixation (30, 32) ou plus, les éléments de fixation (30, 32) ayant une première position d'état et une deuxième position d'état ;
dans lequel un ou plusieurs des éléments de fixation (30, 32) sont montés de manière rotative ;
dans lequel un élément de fixation (30, 32) est monté sur le boîtier (42) par un pivot d'élément de fixation (114) et le pivot d'élément de fixation (114) est mobile par rapport au boîtier (42) ;
dans lequel l'unité de fixation est raccordée à l'élément d'entraînement (66) par un raccord et le raccord est un pivot supplémentaire (102), le pivot supplémentaire (102) étant mobile par rapport au boîtier (42) ;
caractérisé dans lequel le pivot d'élément de fixation (114) a une position distale et une position proximale, le pivot d'élément de fixation (114) étant reçu de manière coulissante dans le boîtier (42) et/ou dans lequel le pivot d'élément de fixation (114) fournit le pivot (114) autour duquel un élément de fixation (30, 32) tourne entre la première position d'état et la deuxième position d'état lorsque le pivot d'élément de fixation (114) est à une position d'extrémité distale par rapport au boîtier (42).

2. Un instrument (20) selon la revendication 1, dans lequel les éléments de fixation (30, 32) ne tournent pas autour de pivots d'élément de fixation (114) lorsqu'ils sont à une position plus proximale par rapport au boîtier (42).

3. Un instrument (20) selon la revendication 1 ou la revendication 2, dans lequel le pivot supplémentaire (102) a une position distale et une position proximale et une position intermédiaire, le pivot supplémentaire (102) étant reçu de manière coulissante dans le boîtier (42) et
dans lequel le pivot supplémentaire (102) entraîne la rotation des éléments de fixation (30, 32) lors de la transition de la position intermédiaire à la position distale par rapport au boîtier.

4. Un instrument (20) selon la revendication 3, dans lequel la position distale est définie par la butée du pivot supplémentaire (102) avec une extrémité distale d'un organe de contrainte sur le boîtier (42).

5. Un instrument (20) selon n'importe quelle revendication précédente, dans lequel les éléments de fixation (30, 32) réalisent une transition de la première position d'état à la deuxième position d'état, la transition inclut une première phase de transition et la transition inclut une deuxième phase de transition, les éléments de fixation (30, 32) restant dans la deuxième position d'état pendant la deuxième phase de transition.

6. Un instrument (20) selon n'importe quelle revendication précédente, dans lequel les extrémités distales (38) des éléments de fixation (30, 32) sont davantage espacées en rotation dans la première position d'état que dans la deuxième position d'état.

7. Un instrument (20) selon n'importe quelle revendication précédente, dans lequel un ou plusieurs des éléments de fixation (30, 32) sont des bras (30, 32) et un ou plusieurs des bras (30, 32) se subdivisent pour donner deux sous-bras (34a, 34b, 36a, 36b) ou plus et/ou dans lequel les éléments de fixation (30, 32) sont pourvus d'une ou de plusieurs saillies (40) au niveau de ou vers leurs extrémités distales (38), les une ou plusieurs saillies (40) sur un élément de fixation (30, 32) étant inclinées vers une ou plusieurs des saillies (40) sur un autre élément de fixation (30, 32) et/ou dans lequel un ou plusieurs des éléments de fixation (30, 32) sont raccordés à un composant de sollicitation (80) et le composant de sollicitation (80) sollicite les éléments de fixation (30, 32) vers la deuxième position d'état.

8. Un instrument (20) selon n'importe quelle revendication précédente, dans lequel l'extrémité proximale de boîtier (22) fournit une surface d'impact (150) et/ou dans lequel l'extrémité distale de boîtier (24) fournit une surface de butée pour un composant chirurgical.

9. Un instrument (20) selon n'importe quelle revendication précédente, dans lequel une interface utilisateur (48) est raccordée de manière opérationnelle à l'élément d'entraînement (66) et la rotation de l'interface utilisateur (48) provoque un mouvement longitudinal de l'élément d'entraînement (66) et de l'unité de fixation.

10. Un instrument (20) selon n'importe quelle revendication précédente, dans lequel l'unité d'entraînement (48) est sollicitée vers l'extrémité proximale (22) du boîtier (42) ou est sollicitée vers l'extrémité distale (24) du boîtier (42) et/ou dans lequel l'élément d'entraînement (66) a une première position d'état et une deuxième position d'état et l'extrémité proximale de l'élément d'entraînement (66) est plus éloignée de l'extrémité proximale de boîtier (22) dans la première position d'état que dans la deuxième position d'état.

11. Un kit, le kit comprenant :
l'instrument chirurgical (20) selon la revendication 1 ; et
un ou plusieurs composants chirurgicaux avec lesquels l'instrument chirurgical (20) se met en prise, le composant chirurgical comprenant un ou plusieurs emplacements de mise en prise ;
dans lequel l'instrument chirurgical (20) est conçu pour se mettre en prise avec le composant chirurgical.

12. Un kit selon la revendication 11, le kit incluant un impacteur et/ou un maillet et/ou le kit incluant une série de composants chirurgicaux d'essai (303) qui sont différents les uns des autres quant aux dimensions d'une ou de plusieurs pièces des composants chirurgicaux d'essai (303) et/ou quant à la géométrie d'une ou de plusieurs pièces des composants chirurgicaux d'essai (303).
